(19) **Europäisches Patentamt / European Patent Office / Office européen des brevets**

(11) **EP 3 498 172 A1**

(12) **EUROPEAN PATENT APPLICATION**
published in accordance with Art. 153(4) EPC

(43) Date of publication:
19.06.2019 Bulletin 2019/25

(51) Int Cl.:
*A61B 6/03* (2006.01)    *A61B 34/10* (2016.01)
*A61B 34/20* (2016.01)

(21) Application number: 17839089.4

(22) Date of filing: 27.06.2017

(86) International application number:
PCT/JP2017/023556

(87) International publication number:
WO 2018/030015 (15.02.2018 Gazette 2018/07)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 08.08.2016 JP 2016155925

(71) Applicant: Kyoto University
Kyoto-shi, Kyoto 606-8501 (JP)

(72) Inventors:
• NAKAO Megumi
Kyoto-shi
Kyoto 606-8501 (JP)
• CHEN Fengshi
Kyoto-shi
Kyoto 606-8501 (JP)

(74) Representative: Hoffmann Eitle
Patent- und Rechtsanwälte PartmbB
Arabellastraße 30
81925 München (DE)

(54) **RESECTION PROCESS ESTIMATION DEVICE AND RESECTION PROCESS NAVIGATION SYSTEM**

(57) A dissection process estimation device is configured from: a surgery process case database storing case data of surgery processes describing information relating to dissection surfaces corresponding to dissection sites of target organs and progress of dissection; a standard dissection process generation unit that reads out case data corresponding to a site to be dissected designated by a user from the surgery process case database and generates a standard dissection surface and a standard dissection process based on plural pieces of read case data; an anatomical reference point extraction unit that extracts an anatomical reference point from patient-specific three-dimensional medical image data; and a patient-specific dissection process estimation unit that performs matching of the standard dissection surface and the standard dissection process with the patient-specific three-dimensional medical image data and estimates a patient-specific dissection surface and a patient-specific dissection process.

FIG.1

EP 3 498 172 A1

## Description

Technical Field

The present invention relates to a device that automatically estimates a dissection process specific to an organ structure of a patient and a navigation system that provides an estimated dissection process.

Background Art

It is extremely important for a medical professional including a doctor and others (hereinafter, referred to as "medical professional") to grasp a dissection plan corresponding to an internal structure of a site to be dissected prior to a surgery. The dissection plan is determined in accordance with a site to be dissected of an organ, an internal structure of a site to be dissected, an operator's experiences, and so forth. For example, a scalpel (not only including a scalpel, but also including other dissection instruments, and, hereinafter referred to as "scalpel") is moved along an important structure, such as blood vessels inside an organ, when the scalpel reaches a predetermined depth and the structure inside the organ can be confirmed, movement of the scalpel is stopped at that time, and then the scalpel is put to another site to be moved to the predetermined depth. On this occasion, regarding the movement of the second scalpel, in a method, the scalpel is put into a surface of the organ on an opposite side of the site that has been dissected first, and in another method, the scalpel is put in a direction slightly different from the first moving direction of the scalpel.

As described above, the dissection plan is carried out through combinations of many factors; among them, it is very important to estimate structures of blood vessels and tumors inside an organ and a three-dimensional positional relationship thereof from the outside of the organ, to thereby determine a site to first start the dissection. When the site to start the dissection is inappropriate, a medical professional is required to put a scalpel again into a most appropriate site; however, unnecessary dissection by repeated insertion of the scalpel imposes a strain on the organ.

Moreover, when there is an error in a progressing direction or depth of progress in the dissection, it is impossible to confirm an anatomical structure inside the organ (for example, a vessel structure of blood vessels, lymph vessels or others) that serves as an important index for progress of dissection, to thereby constitute a hindrance to subsequent progress of dissection. In addition, if the order of dissection is inappropriate (for example, when a scalpel is put into an inappropriate site first), it is not only failing to make a margin to a tumor, but also causing undesired bleeding; as a result, unnecessary dissection is required.

Therefore, for the purpose of supporting to grasp interior of an organ by medical professionals, currently, various kinds of software using image processing techniques or visualization techniques are widely utilized. For example, Patent Document 1 describes a surgical support device that presents a dissection method considering, not only a position of an abnormal region existing in a target organ, but also a positional relationship between the target organ and other organs positioned to surround thereof (refer to paragraph [0010]). Specifically, Patent Document 1 describes a technique that provides a dissection method to minimize a volume to be dissected, a dissection method to minimize a surface area to be dissected, and a dissection method to minimize a distance of a site to be dissected from a surface.

Moreover, Patent Document 2 describes an image diagnosis support technique that enables image simulation considering a degree of deformation of an organ at the time of surgery (refer to paragraph [0006]). Specifically, Patent Document 2 describes a technique that computes in advance an arbitrary dissection pattern for a tetrahedron model constituted by tetrahedron blocks (so-called polygons) in each of which adjacent vertexes are connected to each other, to thereby provide image simulation in accordance with designation of a dissection position by a medical professional.

Citation List

Patent Literature

Patent Document 1: Japanese Patent Application Laid-Open Publication No. 2013-154037
Patent Document 2: Japanese Patent Application Laid-Open Publication No. 2014-176425

Summary of Invention

Technical Problem

The technique described in Patent Document 1 can present candidates for a partial curved surface of the abnormal region from plural viewpoints; however, which of the candidates of the partial curved surface is a standard partial curved surface is unknown. Moreover, in the technique, the candidates for the partial curved surface are known, but, regarding a selected partial curved surface, nothing is shown about in what order to dissect the organ.

On the other hand, the technique described in Patent Document 2 requires calculation of all assumed tetrahedral division patterns in advance regarding all the polygons constituting the target organ; therefore, there is a problem of enormously large calculation load. Moreover, in the technique, it is necessary that a medical professional sequentially designates all vertex positions of each polygon positioned at a site to be dissected. That is, in the method, simulation does not proceed unless the medical professional provides points in succession. Moreover, the technique is absolutely created for each patient on the basis of polygons, and application thereof to medical images is not assumed. In addition, a created polygon model is specific to each individual patient, and application thereof to some other patient is not assumed. Therefore, the technique described in Patent Document 2 remains at a use for training on the dissection process.

In other words, any of the documents does not consider a scheme capable of automatically presenting a dissection process that provides information about into which site to put a scalpel, in what direction to move the scalpel, how deep the moving scalpel is, in what order to put the scalpel, and so forth, without successive designation inputs by a user.

Solution to Problem

This specification includes plural means for solving the above-described problems, and as a specific example thereof, there are means in the following items 1 to 20.

Item 1

A dissection process estimation device including:

a surgery process case database that stores case data of a surgery process describing a dissection surface corresponding to a dissection site of a dissection target organ and information about progress of dissection;
a standard dissection process generation unit that reads case data corresponding to a dissection site designated by a user from the surgery process case database, and generates a standard dissection surface and a standard dissection process based on plural pieces of case data that have been read;
an anatomical reference point extraction unit that extracts anatomical reference points from patient-specific three-dimensional medical image data; and
a patient-specific dissection process estimation unit that performs matching of the standard dissection surface and the standard dissection process with the patient-specific three-dimensional medical image data, and estimates a patient-specific dissection surface and a patient-specific dissection process.

Item 2

The dissection process estimation device described in Item 1, further including:
a dissection point acceptance unit that accepts input of one or plural dissection points to a patient-specific three-dimensional medical image displayed on a display.

Item 3

The dissection process estimation device described in Item 1, wherein,
as preprocessing of the matching, the patient-specific dissection process estimation unit performs:

(i) an enlarging or reducing process that corrects difference in a size of an organ captured on the patient-specific three-dimensional medical image data, the difference being caused by difference in an image capturing condition;
(ii) a distortion correction process that corrects difference between a size of the organ that is specific to a patient and a size of a standard organ; and
(iii) all, a combination or any one of the processes.

Item 4

The dissection process estimation device described in Item 2, wherein the patient-specific dissection process estimation unit estimates the patient-specific dissection process based on the standard dissection process, the anatomical reference points extracted from the patient-specific three-dimensional medical image data, and the dissection points accepted from the user.

Item 5

The dissection process estimation device described in Item 4, wherein the patient-specific dissection process estimation unit calculates the dissection surface passing by near the one or plural dissection points designated by the user as a solution of a minimization problem that assumes positions of the dissection points to be a constraint.

Item 6

The dissection process estimation device described in Item 2, wherein, when the standard dissection surface and the standard dissection process are generated by combining individual pieces of the case data stored in the surgery process case database, the standard dissection process generation unit performs weighting of the pieces of case data to be combined to obtain a combination most approximating the anatomical reference points extracted from the patient-specific three-dimensional medical image data and the dissection points accepted from the user.

Item 7

The dissection process estimation device described in Item 1, wherein
the dissection surface is constituted by a partial curved surface generated in accordance with individual progress of dissection, and
the dissection process provides an order of dissection corresponding to the partial curved surface.

Item 8

The dissection process estimation device described in Item 4, wherein the patient-specific dissection process estimation unit estimates a partial curved surface passing by any of the dissection points based on the standard dissection surface and the standard dissection process.

Item 9

The dissection process estimation device described in Item 8, wherein the patient-specific dissection process estimation unit estimates the partial curved surface by taking into consideration an anatomical structure of the anatomical reference points.

Item 10

The dissection process estimation device described in Item 2, wherein the dissection point acceptance unit accepts input of the one or plural dissection points, and simultaneously, accepts a dissection order of the inputted dissection points.

Item 11

The dissection process estimation device described in Item 10, wherein the patient-specific dissection process estimation unit associates positions and input order of the one or plural dissection points inputted to the patient-specific three-dimensional medical image data with vertexes of each mesh constituting a standard organ, and records the positions and the input order in the surgery process case database.

Item 12

The dissection process estimation device described in Item 1, wherein, when the user designates one or plural dissection points that are specific to a patient, the dissection process estimation device functions as a dissection process navigation system that presents a most suitable dissection surface and dissection process assuming the dissection points to be a constraint to the user.

Item 13

The dissection process estimation device described in Item 1, wherein, when the user designates one or plural dissection points and dissection order that are specific to a patient, the dissection process estimation device functions as a dissection process navigation system that presents a most suitable dissection surface and dissection process assuming the dissection points and input order to be a constraint to the user.

Item 14

The dissection process estimation device described in Item 2, wherein the patient-specific dissection process estimation unit further includes a processing function of associating the standard dissection surface with vertex information of the patient-specific three-dimensional medical image data corresponding to the dissection points designated by the user.

Item 15

The dissection process estimation device described in Item 2, wherein the patient-specific dissection process estimation unit further includes a processing function of associating the patient-specific dissection process and vertex information of the patient-specific three-dimensional medical image corresponding to the dissection points designated by the user with vertex information of a standard organ.

Item 16

The dissection process estimation device described in Item 1, wherein, with progress of the estimated patient-specific dissection process, the patient-specific dissection process estimation unit transparently displays a part or all of the anatomical reference points to be superimposed on the patient-specific three-dimensional medical image.

Item 17

A surgery process case database that stores case data of a surgery process describing a dissection surface corresponding to a dissection site of a dissection target organ and information about progress of dissection.

Item 18

A dissection process navigation system including:

a surgery process case database that stores case data of a surgery process describing a dissection surface corresponding to a dissection site of a dissection target organ and information about progress of dissection;
a standard dissection process generation unit that reads case data corresponding to a dissection site designated by a user from the surgery process case database, and generates a standard dissection surface and a standard dissection process based on a plurality of pieces of case data that have been read;
an anatomical reference point extraction unit that extracts anatomical reference points from patient-specific three-dimensional medical image data;
a patient-specific dissection process estimation unit that performs matching of the standard dissection process with the patient-specific three-dimensional medical image data, and estimates a patient-specific dissection process; and
a display device that displays the patient-specific dissection process estimated in the patient-specific dissection process estimation unit.

Item 19

The dissection process navigation system described in Item 18, wherein, when the user designates one or plural dissection points that are specific to a patient, the display device presents a most suitable dissection surface and dissection process assuming the dissection points to be a constraint to the user.

Item 20

The dissection process navigation system described in Item 18, wherein, when the user designates one or plural dissection points and input order of the respective dissection points that are specific to a patient, the display device presents a most suitable dissection surface and dissection process assuming the dissection points and input order to be a constraint to the user.

Advantageous Effects of Invention

According to the present invention, it is possible to automatically estimate a dissection process that provides information about into which site to put a scalpel, in what direction to move the scalpel, how deep the moving scalpel is, in what order to put the scalpel, and so forth, without successive designation inputting by a user.

Brief Description of Drawings

FIG. 1 is a diagram showing a schematic configuration of a dissection process navigation system related to Example 1;
FIG. 2 is a diagram illustrating a data structure of three-dimensional medical image data;
FIGS. 3A to 3D are diagrams showing images of a standard dissection surface and a standard dissection process;
FIGS. 4A to 4C are diagrams showing estimation processing procedures performed in a patient-specific dissection process estimation unit related to Example 1;
FIG. 5 is a diagram showing a specific example of dissection surfaces and anatomical reference points displayed as a navigation screen related to Example 1;
FIGS. 6A to 6C are diagrams illustrating progress of dissection in the navigation screen;
FIG. 7 is a diagram showing a schematic configuration of a dissection process navigation system related to Example 2;
FIGS. 8A to 8C are diagrams showing estimation processing procedures performed in a patient-specific dissection process estimation unit related to Example 2;
FIG. 9 is a diagram showing a specific example of dissection surfaces and anatomical reference points displayed as a navigation screen related to Example 2;
FIG. 10 is a diagram showing a schematic configuration of a dissection process navigation system related to Example 3;
FIGS. 11A to 11C are diagrams showing estimation processing procedures performed in a patient-specific dissection process estimation unit related to Example 3;
FIG. 12 is a diagram showing a specific example of dissection surfaces and anatomical reference points displayed as a navigation screen related to Example 3;
FIG. 13 is a diagram showing a specific example of newly generated case data; and
FIG. 14 is a diagram showing a specific example of newly generated case data.

Description of Embodiments

Hereinafter, Examples according to the present invention will be described based on the drawings. Note that the present invention is not limited to the Examples to be described as follows and various modifications are available within the scope of the technical idea of the present invention.

(1) Example 1

First, an Example having the simplest device configuration will be described. Specifically, description will be given of a device that, when patient-specific three-dimensional medical image data is provided, navigates the standard dissection site, the direction of progress of the dissection, the depth of the dissection and the order of dissection.

(1-1) Device configuration

(1-1-1) Overall configuration

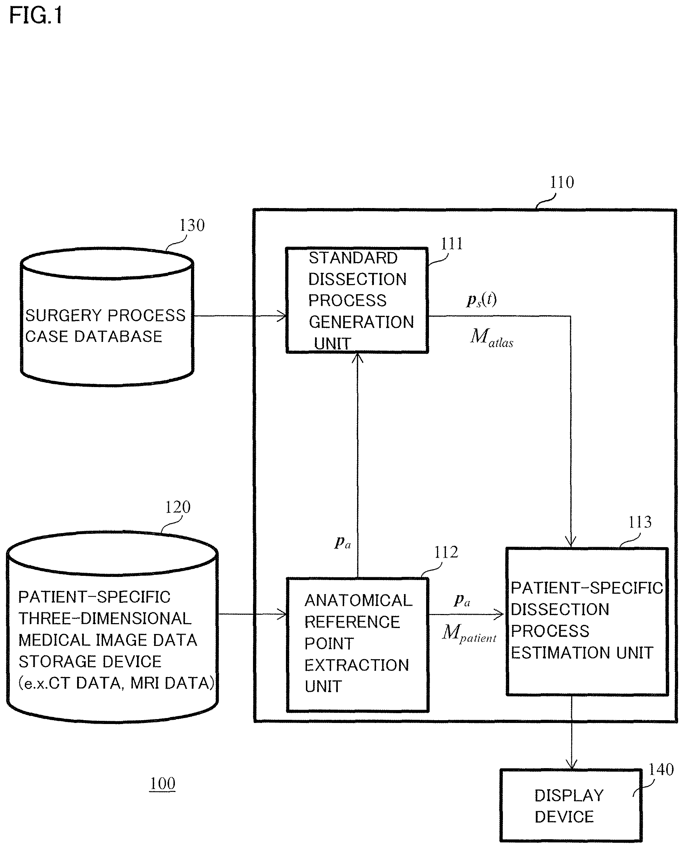

A schematic configuration of a dissection process navigation system 100 in the Example is shown in FIG. 1. The dissection process navigation system 100 includes: a dissection process estimation device 110; a patient-specific three-dimensional medical image data storage device 120; a surgery process case database 130; and a display device 140. The dissection process navigation system 100 may be constructed by using terminals in a medical institution, or may be constructed as a cloud server accessed from terminals in medical institutions via a network. Note that, when the navigation system is configured on a cloud server, the terminals in the medical institutions are used as input/output devices for the cloud server.

The dissection process estimation device 110 is configured with a so-called computer. That is, the dissection process estimation device 110 is configured with a main storage device, an arithmetic device, a control device and an input/output device. The dissection process estimation device 110 implements various kinds of functions, which will be described later, through the execution of the program. Note that a part or all of the functions may be achieved by hardware. The various kinds of functions implemented by the dissection process estimation device 110 will be described later.

Each of the patient-specific three-dimensional medical image data storage device 120 and the surgery process case database 130 is configured with a storage device that stores data to be described later. In the case of the Example, a magnetic disk is used as a storage medium of the storage device. However, other storage media, such as an optical disk, a semiconductor memory or a magnetic tape, may also be used. Each of the patient-specific three-dimensional medical image data storage device 120 and the surgery process case database 130 may be realized as an independent storage device; however, a part or all thereof may be realized as a different storage area in a single storage device. Moreover, these storage devices may be disposed in the same housing with the dissection process estimation device 110, or may be connected thereto via a network. As the network, for example, a dedicated line, the Internet or the like is used. Note that the form of connection may be wired connection or wireless connection.

The patient-specific three-dimensional medical image data storage device 120 is a storage device that stores patient-specific three-dimensional medical image data. The patient-specific three-dimensional medical image data includes, for example, CT (Computed Tomography) image data, MRI (Magnetic Resonance Imaging) image data, PET (Positron Emission Tomography) image data and SPECT (Single Photon Emission Computed Tomography) image data. Note that the three-dimensional medical image data does not need to include all of these image data items, but may include one or more of them.

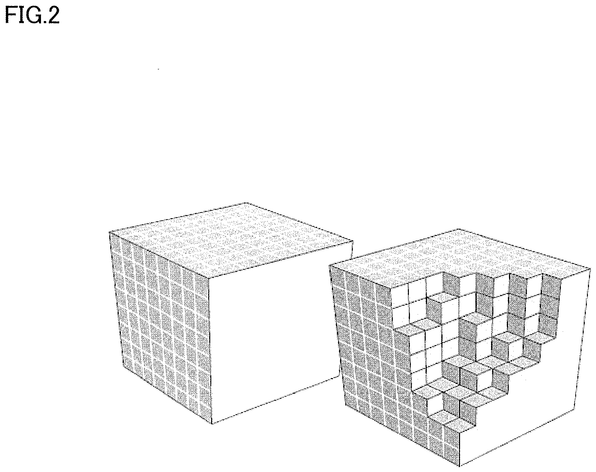

Here, the three-dimensional medical image data is a collection of two-dimensional tomographic images, which is defined by a collection of regular lattice units called voxels. A voxel corresponds to a pixel in a two-dimensional image. In the three-dimensional medical image data, as three-dimensional coordinates are determined, a voxel value is determined. Here, the voxel value corresponding to the three-dimensional coordinate (x, y, z) is represented as I (x, y, z) = {..., (voxel), ...}. In FIG. 2, a data structure of three-dimensional medical image data is shown. The figure on the left is an exterior image of the three-dimensional medical image data, and the figure on the right is an internal structure that is shown by breaking a part of the three-dimensional medical image data. As shown in the figure on the right, inside the three-dimensional medical image data, voxels are disposed without any gaps.

The surgery process case database 130 is a storage device that stores pieces of case data of surgery processes collected in accordance with a dissection site in a dissection target organ. The case data is recorded with respect to a standard organ. The standard organ includes, for example, a liver, a lung, an urinary organ, a respiratory organ, brain and others. The surgery process is configured with a dissection surface and information about progress of dissection. The dissection site refers to a site to be dissected from an organ, which corresponds to, for example, S1 to S8 if the organ is a liver, and a superior lobe, an intermediate lobe, an inferior lobe and so forth if the organ is a lung. In the case of the Example, a dissection surface refers to a cross section of an organ that appears in a final stage of the dissection. Plural partial curved surfaces successively appearing in accordance with progress of dissection by a scalpel lead to reach the dissection surface. Note that the shape of the partial curved surfaces changes in response to the progress of dissection by the scalpel.

The dissection process is information about dissection leading to the dissection surface. The dissection process refers to information about into which site to put a scalpel, in which direction to move the scalpel, how deep the moving scalpel is, in what order to put the scalpel, and so forth. In the case of the Example, in the surgery process case database 130, the dissection surface and the dissection process are stored in association with the standard organ. Note that the dissection surface and the dissection process are associated with the three-dimensional coordinates of the three-dimensional medical image data representing the standard organ. The anatomical structure of the standard organ is known. Therefore, the value I (x, y, z) of each three-dimensional coordinates constituting the case data is represented as {..., (voxel, label), ...} by using a voxel value "voxel" possessed by an medical image and a label value "label" of an anatomical structure label. The label value will be described later.

The display device 140 is connected to the dissection process estimation device 110 and is used to provide a user interface screen. On the user interface screen, for example, a patient-specific three-dimensional medical image or a dissection process estimated by the dissection process estimation device 110 is displayed. As the display device 140, for example, a flat display device, such as an LCD monitor, is used.

(1-1-2) Detailed configuration

The detailed configuration of the dissection process estimation device 110 will be described. The dissection process estimation device 110 provides various kinds of functions through the execution of the program. In the case of the Example, the dissection process estimation device 110 provides the functions corresponding to a standard dissection process generation unit 111, an anatomical reference point extraction unit 112 and a patient-specific dissection process estimation unit 113.

The standard dissection process generation unit 111 reads case data corresponding to an organ and a dissection site, which have been designated beforehand by a medical professional who is a user of the system, from the surgery process case database 130, to thereby generate a standard dissection surface and a standard dissection process Ps(t). The parameter t refers to a time stamp that means information about a degree of progress of dissection or a dissection order.

The standard dissection process generation unit 111 combines the individual pieces of case data stored in the surgery process case database 130 to generate the standard dissection surface and the standard dissection process Ps(t). The standard dissection process generation unit 111 in the Example performs weighting calculation of plural pieces of case data, to thereby generate the standard dissection surface and the standard dissection process Ps(t). On this occasion, the standard dissection process generation unit 111 determines a pair of weights with which the anatomical reference point inside the standard organ generated by the weighting calculation comes closest to an anatomical reference point Pa extracted from the patient-specific three-dimensional medical image data, and uses the dissection surface and the dissection process calculated by use of the pair of weights as "standard dissection surface" and "standard dissection process".

In the Example, standard dissection process data Matlas, which includes the following data structure, is used. The standard dissection process data Matlas is expressed as follows by use of a vertex set V and an element set E.

$$\mathrm{Matlas} = (V, E)$$

V is a set of vertexes in each mesh constituting the three-dimensional medical image data representing the standard organ, and expressed as follows by using the vertex vector vi.

$$V = \{v0, v1, ..., vn\text{-}1\}$$

The vertex vector vi is expressed as follows by using the vertex coordinates (x, y, z), the time stamp "time", the anatomical structure label "label" and attribute information of vertex (isFreezed, isCutpath, isSurface).

$$vi = (x, y, z, \mathrm{time}, \mathrm{label}, \mathrm{isFreezed}, \mathrm{isCutpath}, \mathrm{isSurface})$$

x, y, z are values of the three-dimensional positions of the corresponding vertexes saved as floats.

"time" refers to a time stamp showing in what order the corresponding vertex is dissected, which is given as, for example, integers of [0, n-1]. Note that, to a vertex not to be dissected, these integer values are not allocated, or a value other than these integer values is allocated.

"label" is a label value of an integer showing an anatomical structure and, for example, "0" is given to a superior lobe, "1" is given to an intermediate lobe, "2" is given to an inferior lobe, "3" is given to an artery, "4" is given to a vein, and so forth.

"isFreezed" is attribute information 1, in which "1" is given to a fixed vertex, and "0" is given to a free vertex.

"isCutpath" is attribute information 2, in which "1" is given to a vertex on the dissection surface, and "0" is given to other vertexes.

"isSurface" is attribute information 3, in which "1" is given to a vertex on a surface of an organ, and "0" is given to a vertexes positioned inside the organ.

The element set E provides which vertex constitutes the minimum element of a mesh form as a pair of vertex numbers which are integers. In the case of a triangular mesh, the element set E is expressed by a set of three vertexes. When the minimum element is a tetrahedron, the element set E is expressed by a set of four vertexes, such as, for example, E = {(0, 1, 2, 3), (0, 3, 6, 9), ..., (p, q, r, s), ...}.

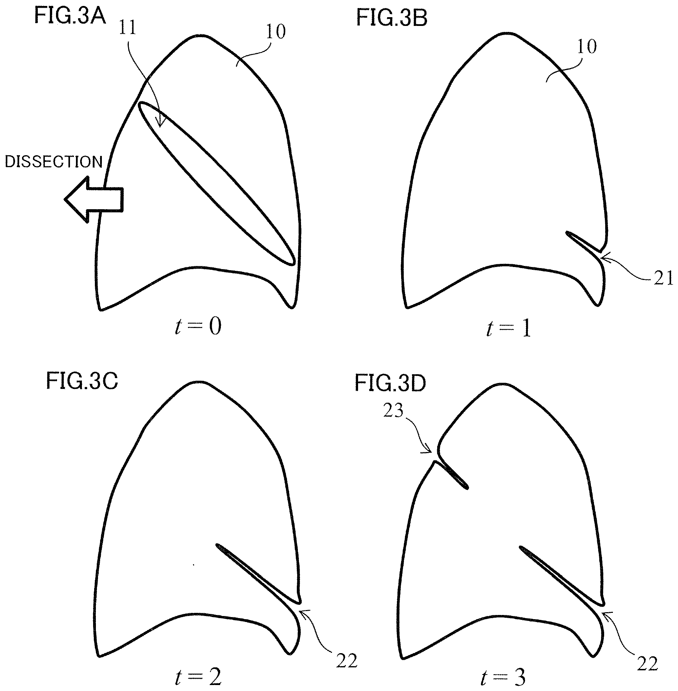

FIG. 3 shows image diagrams of the standard dissection surface 11 and the standard dissection process data Matlas generated by the standard dissection process generation unit 111. FIG. 3 shows a specific example of dissecting a right inferior lobe of a lung, which is the standard organ 10. The four times corresponding to the time stamp t = 0, 1, 2, 3 correspond to the standard dissection processes Ps(0), Ps(1), Ps(2), Ps(3). The time stamp t = 0 (refer to FIG. 3A) corresponds to the time before the dissection is started, and represents a relationship between the standard dissection surface 11 providing a surface planned for dissection to be generated from now on and a site to be dissected (the inferior lobe) from the lung 10. In the case of FIG. 3, the standard dissection surface 11 is represented as a flat surface; however, this is to facilitate understanding, and the standard dissection surface 11 is defined by a free-form surface in actuality.

The respective image diagrams corresponding to time stamp t = 1, 2, 3 (refer to FIGS. 3B, 3C, 3D) represent deformation of the organ when the scalpel cuts through the organ to connect the vertexes, of the vertex set V, provided with the same time stamp. Cuts 21 to 23 in the figures represent information about from which site on the surface of the organ to put the scalpel, in what direction to move the scalpel, how deep to insert the scalpel, and in what order to put the scalpel.

The anatomical reference point extraction unit 112 uses an existing area extraction algorithm and provides a function that extracts tubular structures and lesions present in the dissection site, as the anatomical reference points Pa, from the patient-specific three-dimensional medical image data. For example, based on the brightness distribution in the patient-specific three-dimensional medical image data, the anatomical reference point extraction unit 112 extracts anatomical information about the lung, liver or others, the tubular structures (for example, the blood vessels or lymphatic vessels), the lesions (for example, the tumors), or the like. The anatomical reference point extraction unit 112 provides corresponding label values to the extracted anatomical reference points Pa to generate patient-specific three-dimensional medical image data Mpatient.

Here, the patient-specific three-dimensional medical image data Mpatient is expressed as I (x, y, z) = {..., (voxel, label), ...}, which is the above-described voxel value I (x, y, z) provided with the label value "label" of the anatomical structure label. The patient-specific three-dimensional medical image data Mpatient is represented by the vertex set V+ and the element set E. However, since the vector data here that provides the vertex set V+ does not include the time stamp and the like, the vertex vector v+i is expressed as follows by the vertex coordinates (x, y, z) and the anatomical structure label "label".

$$v{+}i = (x, y, z, label)$$

The patient-specific dissection process estimation unit 113 performs matching of the standard dissection surface and the standard dissection process data Matlas with the patient-specific three-dimensional medical image data Mpatient, to thereby provide a function of estimating a patient-specific dissection surface and dissection process data M'. Note that, as preprocessing of the matching, the patient-specific dissection process estimation unit 113 performs: (i) an enlarging or reducing process for correcting difference in the patient-specific organ sizes caused by different imaging conditions; (ii) a distortion correction for correcting difference between the patient-specific organ size or partial form resulting from individual variability and the size or form of the standard organ; and (iii) all of the above processes, a combination of the above processes, or any one of the above processes. This makes it possible to increase accuracy of the subsequent matching process.

Next, the patient-specific dissection process estimation unit 113 associates each vertex of the voxels constituting the standard organ with each vertex of the voxels constituting the patient-specific three-dimensional medical image data Mpatient, to thereby estimate the patient-specific dissection surface and dissection process data M'. Since the standard dissection surface and the standard dissection process data Matlas are selected so that the anatomical reference point of the standard organ generated by the weighting calculation comes closest to the patient-specific anatomical reference point Pa, the dissection surface and the dissection process data M' that are most appropriate to surgery of the patient are provided.

When estimation of the patient-specific dissection surface and dissection process data M' is finished, the patient-specific dissection process estimation unit 113 generates a navigation screen based on the estimated dissection surface and dissection process data M' and displays thereof on the screen of the display device 140. On the navigation screen, into which site to put a scalpel, in which direction to move the scalpel, how deep the moving scalpel is, in what order to put the scalpel, and so forth are presented in chronological order. Accordingly, a medical professional can confirm the dissection surface and dissection process suitable to the individual patient without inputting nothing other than the patient's individual three-dimensional medical image data Mpatient.

(1-2) Processing operation

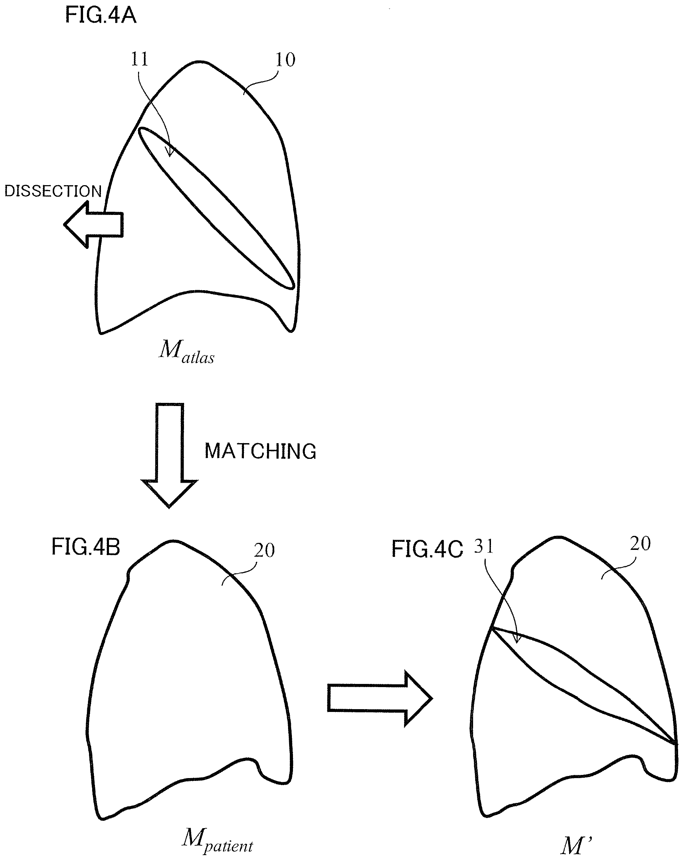

An overview of processing operation of the dissection process navigation system 100 will be described by using FIG. 4.

First, the dissection process estimation device 110 uses the standard dissection process generation unit 111 to calculate the standard dissection surface 11 and the standard dissection process data Matlas for the standard organ 10. As described above, the standard dissection process data Matlas includes a series of information related to progress of dissection leading to the standard dissection surface 11. In FIG. 4A, only the standard dissection surface 11 is shown.

Next, the patient-specific dissection process estimation unit 113 obtains the three-dimensional medical image data Mpatient calculated for the patient's organ (the lung 20) from the anatomical reference point extraction unit 112, and performs matching thereof with the standard dissection process data Matlas (refer to FIG. 4B). In the matching process, the dissection process estimation device 110 aligns the vertex set V = {v0, v1, ..., vn-1} of the n vertexes in the standard dissection process data Matlas with the vertex set V+ = {v+0, v+1, ..., v+m-1} of the m vertexes in the patient-specific three-dimensional medical image data Mpatient.

vi = (x, y, z, time, label, isFreezed, isCutpath, isSurface, ...)
where i = [0, n-1]

$$v+j = (x', y', z', label')$$

where j = [0, m-1]

The dissection process data M' after being aligned with the patient-specific vertex set V+ is expressed by the following expression.

M' = (x*, y*, z*, time, label, isFreezed, isCutpath, isSurface, ...)

As a result, as shown in FIG. 4C, the dissection process estimation device 110 is able to calculate the patient-specific dissection surface 31 for the patient's organ 20.

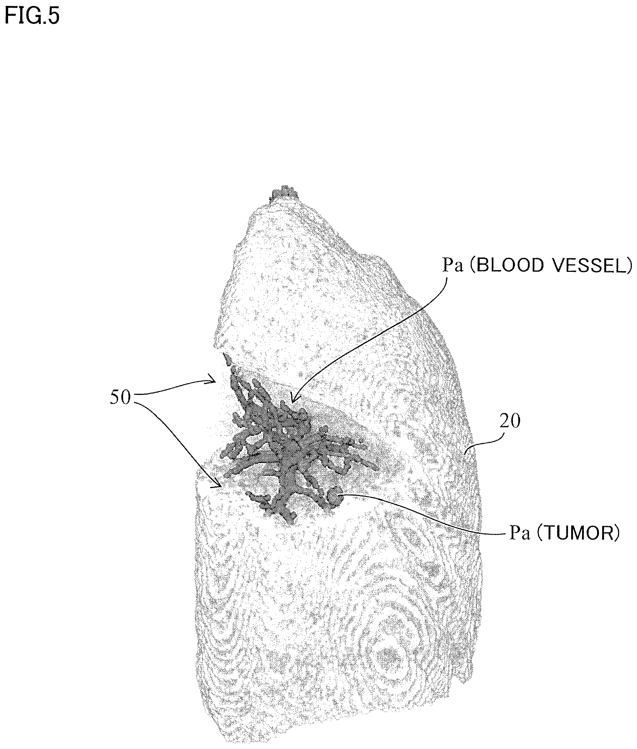

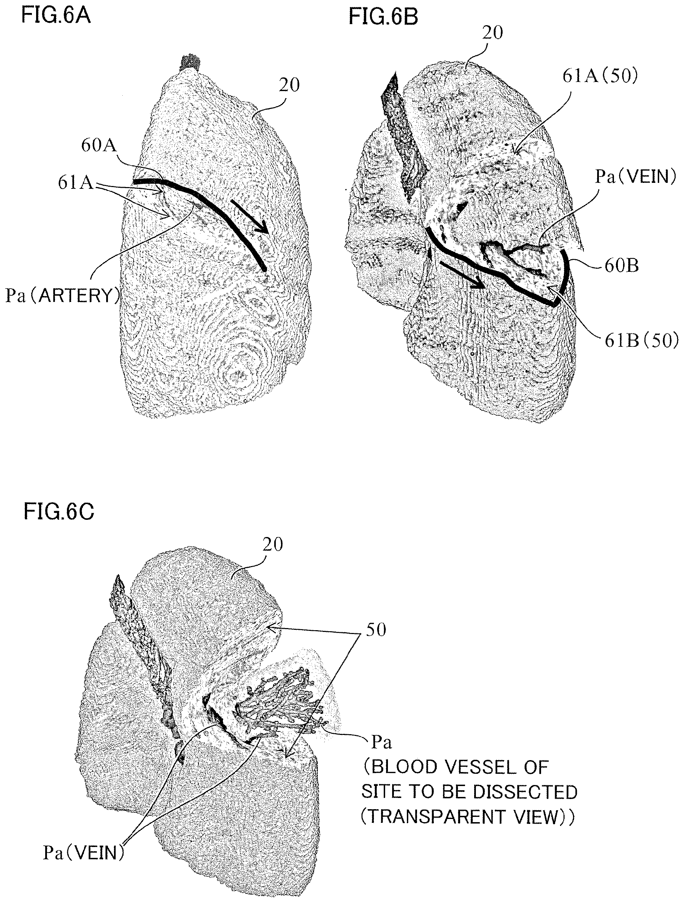

Conceptual examples of the navigation screen are shown in FIGS. 5 and 6. Different from the specific examples in FIGS. 3 and 4, FIGS. 5 and 6 show a case of dissecting an intermediate lobe of the lung 20. FIG. 5 is an initial screen in the navigation screen. FIG. 5 shows a dissection surface 50 estimated for the patient-specific three-dimensional medical image data Mpatient and an anatomical structure inside the organ including partial Pa among the anatomical reference points Pa, transparently, to be referenced in the dissection process that will be described later. Note that, in the specific example in FIG. 5, as an instance of the display mode, only the anatomical structure of the site planned to be dissected from an organ (in this specific example, the lung 20) is transparently shown; however, it is also possible to transparently display the anatomical structure of only the site to be left or of the whole organ. From the display, a medical professional can grasp the anatomical structure inside the organ, such as positions of the arteries, the veins and the tumors, which cannot be observed from the outside of the organ. However, only by the initial screen, it is impossible to obtain information about into which site to put a scalpel, in which direction to move the scalpel, how deep the moving scalpel is, and in what order to put the scalpel.

The patient-specific dissection process estimation unit 113 displays the navigation screens shown in FIGS. 6A to 6C in order of time. Each of FIGS. 6A to 6C corresponds to a point on a time axis, and in actuality, partial curved surfaces 61A, 61B and the shape of the organ in accordance with the movement of the scalpel are displayed to be continuously changed. FIG. 6A is a diagram viewing the lung 20 from the front thereof. The diagram shows a state in which the partial curved surface 61A appears by movement of a blade of the scalpel inserted into the organ along a curved line 60A indicated by a bold line. In other words, the curved line 60A indicates positions on the surface of the organ to put the scalpel. Note that, on the navigation screen, the curved line 60A is colored in a specific color for purpose of visually recognizing the dissection location with ease. Pieces of the three-dimensional medical image data of the vertexes corresponding to the curved line 60A are provided with the same time stamp. The arrow in the diagram indicates the direction to move the scalpel. From the displayed contents in FIG. 6A, it is understood that the scalpel is to be inserted to a depth at which the artery can be observed, the artery being the anatomical reference point Pa.

The curved line 60B in FIG. 6B shows a site to put the second scalpel. The second scalpel is put from the rear side of the lung 20 and moved forward. In FIG. 6B, also, the curved line 60B is colored in a specific color for purpose of easily performing visual recognition of positions on the surface of the organ into which the scalpel is put. The medical professional who has confirmed the display in FIG. 6B can understand that the second scalpel should be moved forward from the rear side of the lung along the curved line 60B. Moreover, it is shown that the scalpel is to be inserted to a depth at which the vein can be observed, the vein being the anatomical reference point Pa. The partial curved surface 61B appears by the movement of the second scalpel. FIG. 6C shows a state in which cross sections of blood vessels on the dissection surface 50 newly appear by further dissection carried out after the progress of dissection by the second scalpel. Note that, by connecting the partial curved surface 61A appearing by the progress of dissection by the first scalpel and the partial curved surface 61B appearing by the progress of dissection by the second scalpel in a bowl shape, the portion sandwiched by the two partial curved surfaces 61A and 61B is dissected from the organ.

In FIG. 6C, the anatomical structure of the veins or others appearing on the dissection surface 50 is shown, as well as the anatomical structure of the blood vessels or others in the site to be dissected from the organ (in the specific example, the lung 20) is transparently shown. By these displays, the medical professional can grasp information necessary to surgery proceedings, such as cross sections of blood vessels appearing in the site to be left in the organ or cross sections of blood vessels contained in the site to be dissected. Further, according to the display in FIG. 6C, the anatomical structure inside the site to be dissected, which is not observed from the outside in a real organ, can be transparently confirmed, and therefore, it is also possible to confirm whether or not a tumor or the like that should be cut off has been cut off. Note that, also in FIGS. 6A to 6C, similar to the aforementioned FIG. 5, it is also possible to transparently display the anatomical structure only of the site to be left or of the whole organ.

By the way, the patient-specific dissection process estimation unit 113 grasps the positional relationship between the curved lines 60A, 60B on which the scalpel moves and the patient-specific three-dimensional medical image data Mpatient. Therefore, the navigation screen displays the patient-specific shape of the organ (the lung 20) to be deformed in accordance with the depth that the scalpel reaches and the movement of the scalpel along the curved lines 60A and 60B. Since information about the vertex set V+ of each mesh given as the patient-specific three-dimensional medical image data Mpatient can be used for deformation of the organ (the lung 20) here, the calculation amount is small as compared to Patent Document 2 that uses polygons. Therefore, it is possible to display changes in the internal structure or shape appearing on the partial curved surfaces 61A and 61B in real time.

(1-3) Effects

By using the above-described dissection process navigation system 100, the medical professional can receive presentation of navigation screens about the dissection surface 50 and the dissection process suitable to the patient's organ (the lung 20) by only inputting the patient-specific three-dimensional medical image data. In other words, without successive designation inputting of the dissection points by a medical professional, who is a user, it is possible to automatically estimate the dissection surface 50 and the dissection process. As a result, the medical professional can automatically confirm the surgery process based on textbook-style information, in particular, procedures about into which site to put a scalpel, in which direction to move the scalpel, how deep the moving scalpel is, in what order to put the scalpel, and so forth. Moreover, by using the system, it is possible to visualize, with the progress of dissection, anatomical structures of blood vessels or others or deformation of the organ expected to appear with the progress of dissection. On that occasion, not only the anatomical structures directly appearing on the dissection surface, but also the anatomical structures inside the organ or inside the site to be dissected can be transparently visualized in accordance with selection by the medical professional. As a result, the medical professional can perform the surgery with reference to chronological visualization results.

(2) Example 2

(2-1) Device configuration

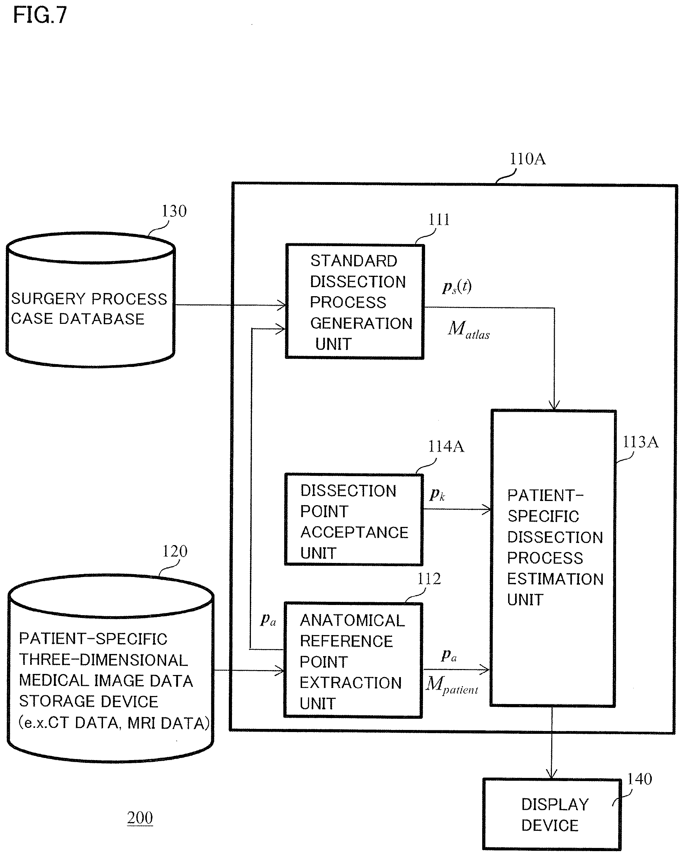

A schematic configuration of a dissection process navigation system 200 in the Example is shown in FIG. 7. In FIG. 7, components corresponding to those of FIG. 1 are provided with same reference signs. The dissection process navigation system 200 includes: the dissection process estimation device 110A; the patient-specific three-dimensional medical image data storage device 120; the surgery process case database 130; and the display device 140.

The dissection process estimation device 110A of the Example differs from the dissection process estimation device 110 of Example 1 (FIG. 1) in the point of including a dissection point acceptance unit 114A. The dissection point acceptance unit 114A provides a function of accepting input of one or plural dissection points Pk by a medical professional with respect to the patient-specific three-dimensional medical image data displayed on the screen of the display device 140. The function is prepared for the purpose of approximating the finally-estimated dissection surface and dissection process to an image held by the medical professional.

In the case of the Example, input order of the dissection points Pk is meaningless. Input of the dissection points Pk is performed through positional input of a pointer positioned on the surface of the three-dimensional medical image data. The number of inputs of the dissection points Pk is not limited. Accordingly, the number of inputs may include one.

When the standard dissection surface 11 and the standard dissection process are generated by combining individual pieces of case data stored in the surgery process case database 130, the standard dissection process generation unit 111 in the Example performs weighting of case data to be combined so that a combination most approximating the anatomical reference points Pa extracted from the patient-specific three-dimensional medical image data and the dissection points Pk accepted from the user can be obtained.

The patient-specific dissection process estimation unit 113A in the Example estimates the patient-specific dissection surface and dissection process taking the positional information of the dissection points Pk inputted by a medical professional into consideration, too. For example, the patient-specific dissection process estimation unit 113A includes a function of deforming, in real time, the patient-specific dissection surface obtained by matching with the standard dissection surface so that the patient-specific dissection surface passes near the dissection point Pk {p0, p1, ..., pn-1}.

(2-2) Processing operation

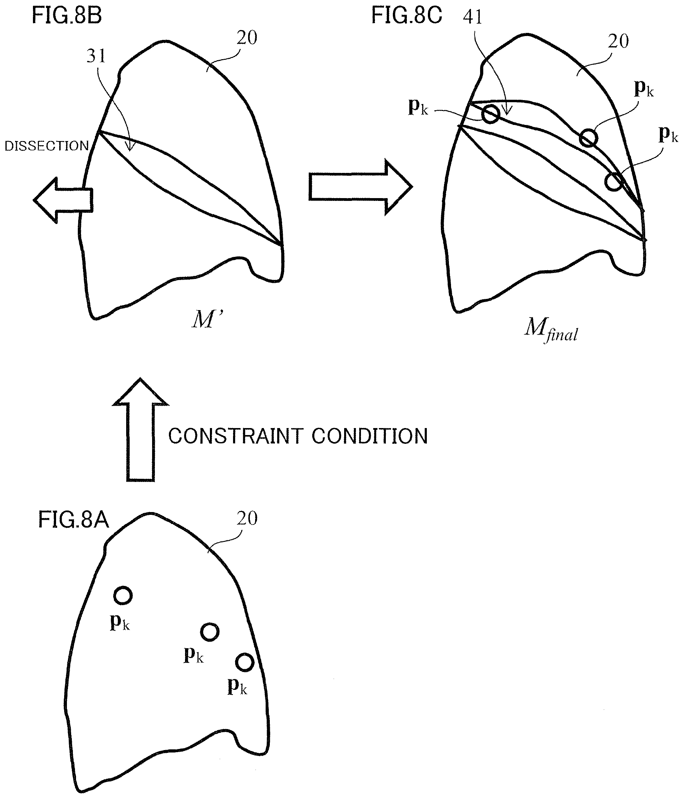

An overview of processing operation of a dissection process navigation system 200 will be described by using FIGS. 4 and 8.

First, the patient-specific dissection process estimation unit 113A uses the standard dissection process generation unit 111 to calculate the standard dissection surface 11 (refer to FIG. 4A) and the standard dissection process data Matlas for the standard organ 10. The operation is the same as that of Example 1. In other words, as shown in FIG. 4C, the standard dissection surface 11 and the standard dissection process data Matlas are applied to the patient-specific three-dimensional medical image data Mpatient, to thereby calculate a patient-specific dissection surface 31 and dissection process data M'. FIG. 8B shows the state.

In the case of the Example, the medical professional inputs the dissection points Pk defining the positions through which the scalpel passes in his/her judgment via the dissection point acceptance unit 111A (refer to FIG. 8A). Therefore, the patient-specific dissection process estimation unit 113A provides these dissection points Pk as constraints for the above-described patient-specific dissection surface 31 and dissection process data M', and adds modifications so that the patient-specific dissection surface 31 and the dissection process data M' pass by near the dissection points Pk. In the case of FIG. 8A, three dissection points Pk are inputted.

The patient-specific dissection process estimation unit 113A performs modification process by using the calculation expression shown as follows. First, the patient-specific dissection process estimation unit 113A calculates a set of vertexes of the meshes positioned on a modified dissection surface 41 by use of the following expression (1) (the set is indicated by adding ^ above V in the expression).
[Expression 1]

$$\hat{V} = \underset{V}{\mathrm{argmin}} \sum_{i=0}^{n-1} \|L(\hat{v}_i) - L(v_i)\|^2 + \sum_{i=0}^{n-1} \|p_i - v_i\|^2 \quad (1)$$

Here, V is a set of vertexes of meshes positioned on the patient-specific dissection surface 31 and given as V = {v0, v1, ..., vn-1}. Further, vi (i = 0, 1, ..., n-1) is an initial position of the position coordinates, and vi^ (in the expression, ^ is added above v) is the position coordinates that provides a solution of the least-square method. Note that L(-) denotes Laplacian. Moreover, pi (i = 0, 1, ..., n-1) denotes a positional constraint for i-th vertex. In addition, N(vi) (i = 0, 1, ..., n-1) is a set of vertexes positioned near the i-th vertex. Here, the Laplacian vector at the i-th vertex can be calculated by use of the weight wij as shown in the following expression (2).
[Expression 2]

$$L(v_i) = \sum_{j \in N(v_i)} w_{ij}(v_i - v_j) \qquad (2)$$

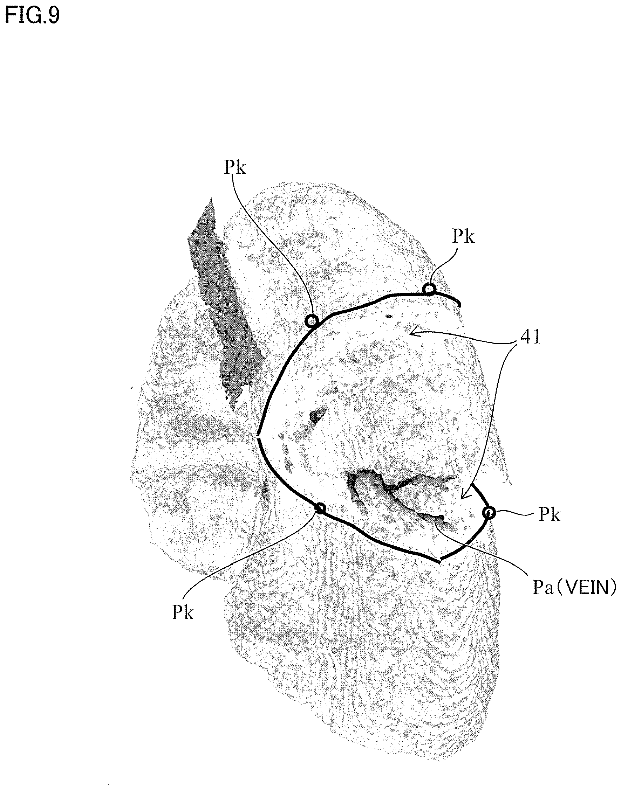

One of the features of the calculation method is to only use the information of vertexes of the meshes positioned on the patient-specific dissection surface 31. Therefore, as compared to Patent Document 2 that needs calculation of all the polygons in a standard model, the required calculation amount is extremely small. As a result, it is possible to calculate the modified dissection surface 41 (refer to FIG. 8C) that includes the three dissection points Pk provided as the constraints in real time. Note that, in the Example, the reason why the modified dissection surface 41 is solved as a minimization problem is that there is a possibility of absence of the dissection surface including all the three dissection points Pk. A conceptual example of the navigation screen is shown in FIG. 9. Different from FIG. 8, FIG. 9 shows a case of dissecting an intermediate lobe of a lung. In FIG. 9, an edge portion of the modified dissection surface 41 is indicated by a broken line. Of course, in the case of FIG. 9, not only the anatomical structures directly appearing on the modified dissection surface 41, but also the anatomical structures inside the organ or inside the site to be dissected may be transparently visualized in accordance with selection by the medical professional.

(2-3) Effects

By using the above-described dissection process navigation system 200, in addition to the effects of Example 1, it is possible to modify the initial cut 21 and the dissection process obtained by application of the standard dissection surface and the standard dissection process to pass the dissection points Pk designated by the medical professional. Note that, in the calculation performed in the modification process, calculation load is small because only the information about vertexes of meshes positioned on the patient-specific dissection surface 31 is used, and the modified dissection surface 41 and the modified dissection process data Mfinal can be calculated in real time.

(3) Example 3

(3-1) Device configuration

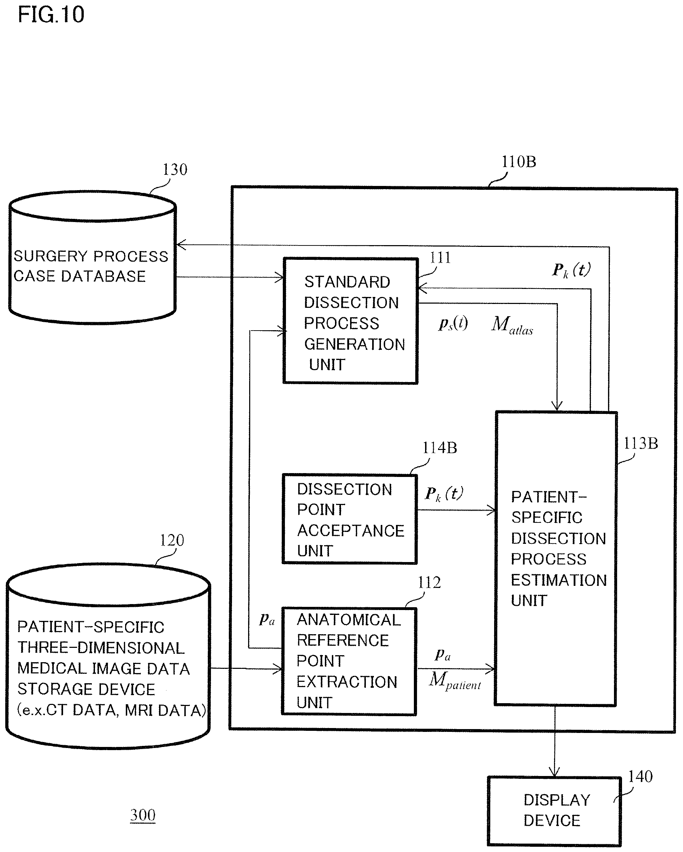

A schematic configuration of a dissection process navigation system 300 in the Example is shown in FIG. 10. In FIG. 10, components corresponding to those of FIG. 7 are provided with same reference signs. The basic configuration of the dissection process navigation system 300 is same as the dissection process navigation system 200 in Example 2.

The features of the Example are the following three: (1) a dissection point acceptance unit 114B also provides the input order of the dissection points Pk to a patient-specific dissection process estimation unit 113B; (2) the patient-specific dissection process estimation unit 113B estimates a modified dissection surface and a modified dissection process suitable to a patient while taking into consideration the inputted positions of the dissection points Pk and the input order; and (3) the patient-specific dissection process estimation unit 113B records the inputted positions of the dissection points Pk and input order thereof to the surgery process case database 130 in association with the vertexes of the meshes constituting the standard organ.

(3-2) Processing operation

In the following, processing operations related to the aforementioned features will be described.

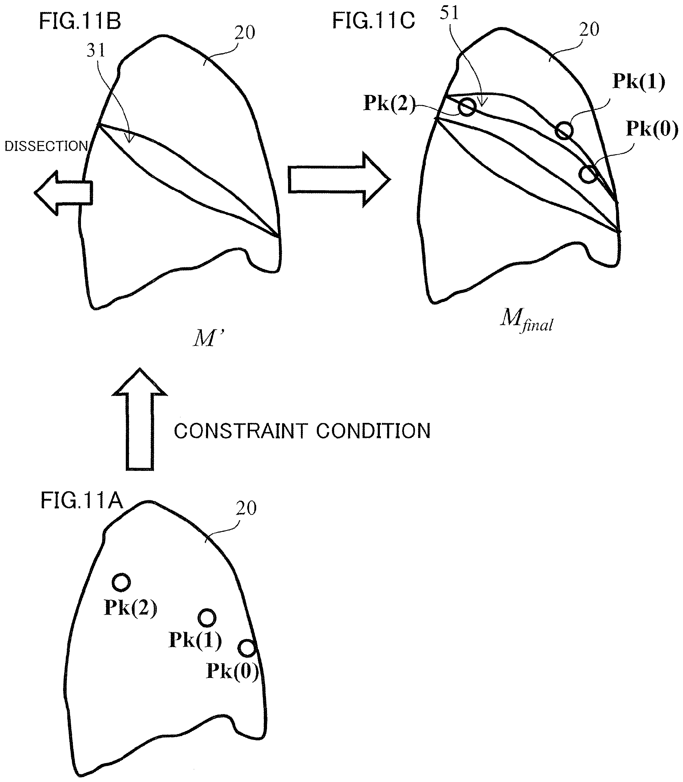

To begin with, the first feature will be described. The dissection point acceptance unit 114B in the Example specifies the input of the dissection point Pk by the medical professional and simultaneously the input order thereof, to thereby output the dissection point Pk and the input order to the patient-specific dissection process estimation unit 113B as the dissection point Pk(t). In FIG. 11, an input example of the dissection point Pk(t) is shown. In FIG. 11A, three dissection points Pk(0) to Pk(2) are inputted. Here, Pk(0) shows the position of the firstly-inputted dissection point, Pk(1) shows the position of the secondly-inputted dissection point, and Pk(2) shows the position of the thirdly-inputted dissection point.

Next, the second feature will be described. The patient-specific dissection process estimation unit 113B in the Example determines whether the input order of the dissection points Pk(0) to Pk(2) matches development of dissection (the order of dissecting an organ with a scalpel) in the standard dissection process, and if matches, the modified dissection surface 51 and the modified dissection process data Mfinal generated by the same method as Example 2 are calculated (refer to FIGS. 11B and 11C). In contrast thereto, when the input order of the dissection points Pk(0) to Pk(2) does not match the development order of dissection in the standard dissection process, the patient-specific dissection process estimation unit 113B, for example, provides information about the input order of the dissection points Pk(0) to Pk(2) to the standard dissection process generation unit 111, and by selectively using the case data that matches the input order of the dissection points Pk(0) to Pk(2), and recalculates the standard dissection surface and the standard dissection process data (refer to FIG. 11B).

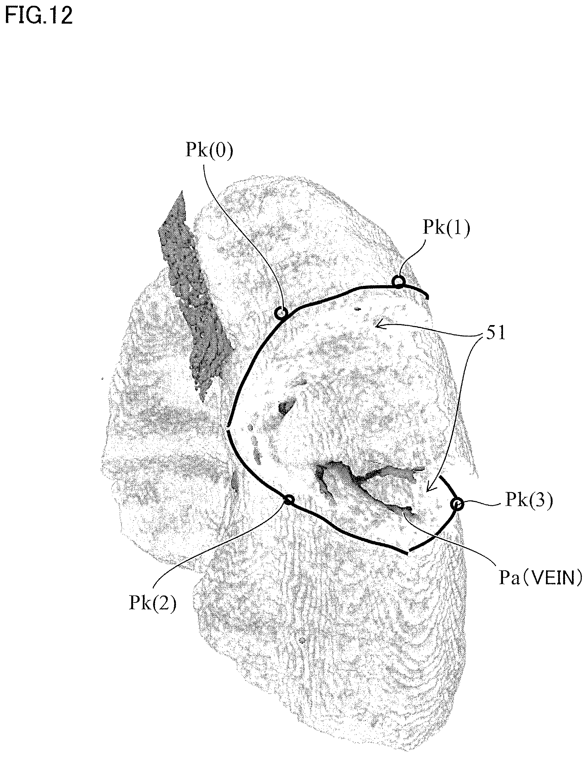

Thereafter, the standard dissection surface and the standard dissection process data Matlas obtained by recalculation are modified in accordance with the positions of the dissection points Pk(0) to Pk(2), and thereby the modified dissection surface 51 and the modified dissection process data Mfinal are generated (refer to FIG. 11C). A specific example of the navigation screen in the Example is shown in FIG. 12. Same as the above-described Examples, FIG. 12 also shows the case of dissecting the intermediate lobe of the lung. In the case of the Example, the modified dissection surface 51 and the modified dissection process data Mfinal matching the input order of the dissection points Pk(0) to Pk(2) by the medical professional are automatically estimated and presented on the screen. Of course, in the case of FIG. 12, not only the anatomical structures directly appearing on the modified dissection surface 51, but also the anatomical structures inside the organ or inside the site to be dissected may be transparently visualized in accordance with selection by the medical professional.

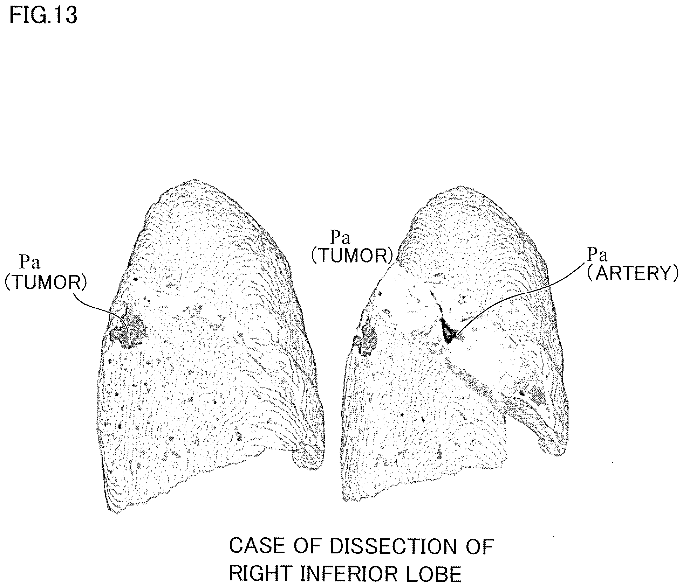

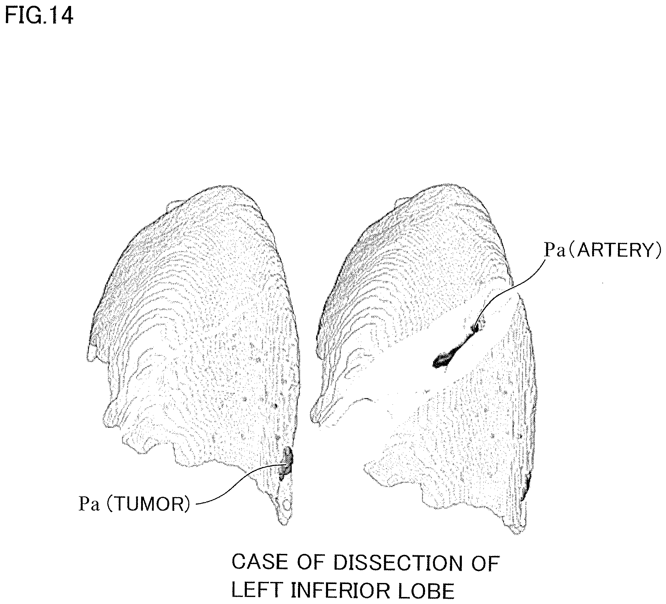

The third feature will be described. This corresponds to a process that associates the information about the positions of the dissection points Pk(t) inputted by the medical professional and the input order with the vertexes of meshes constituting the standard organ and stores thereof. In performing the process, the patient-specific dissection process estimation unit 113B enlarges or reduces the patient-specific three-dimensional medical image data used in the patient-specific dissection process estimation unit 113B to carry out the matching process with the standard organ, and allocates the positions of the matched dissection points Pk(t) and the time stamps to the vertexes of meshes constituting the standard organ. In FIGS. 13 and 14, specific examples of newly registered case data are shown. FIG. 13 shows case data corresponding to dissection of a right inferior lobe, and FIG. 14 shows case data corresponding to dissection of a left inferior lobe.

(3-3) Effects

By using the above-described dissection process navigation system 300, accumulation of case data proceeds, and it is possible to increase estimation accuracy of the standard dissection surface and the standard dissection process. Moreover, it is possible to present the dissection surface and the dissection process close to the dissection order thought by the medical professional as an estimation result. Moreover, by using the system, a representative dissection pattern in each organ, for example, the dissection of the right inferior lobe shown in FIG. 13 or the dissection of the left inferior lobe shown in FIG. 14 is made into a template and stored as the standard dissection process data, and thereby, a surgery text (so-called "Atlas"), which has conventionally been written only on paper, can be constructed as an electronic surgery text. Of course, in the case of FIG. 13 or FIG. 14, not only the anatomical structures directly appearing on the dissection surface, but also the anatomical structures inside the organ or inside the site to be dissected may be transparently visualized in accordance with selection by the medical professional.

Further, if various types of standard dissection process data corresponding to these standard templates are stored in the surgery process case database 130 in advance, the standard dissection process data is aligned with the patient's individual three-dimensional medical image data based on an objective function, and accordingly, it is possible to reproduce a standard dissection method. In other words, the standard dissection method is customized (individualized) and applied to three-dimensional medical image data (an outer shape of an organ, anatomical structures of tumors, blood vessels or others) prepared for a new patient, and it is possible to finally visualize a patient-specific most suitable dissection method and reproduce thereof.

Reference Signs List

10: Standard organ
11: Standard dissection surface
20: Patient's organ
21, 22, 23: Cut
31: Patient-specific dissection surface
41, 51: Modified dissection surface
50: dissection surface applied to patient-specific three-dimensional medical image data (standard dissection surface)
60A, 60B: Curved line (site on organ's surface to be dissected)
61A, 61B: Partial curved surface (surface appearing by dissection with scalpel)
100, 200, 300: Dissection process navigation system
110, 110A, 110B: dissection process estimation device
111: Standard dissection process generation unit
112: Anatomical reference point extraction unit
113, 113A, 113B: Patient-specific dissection process estimation unit
114A, 114B: Dissection point acceptance unit
120: Patient-specific three-dimensional medical image data storage device
130: Surgery process case database
140: Display device
Pa: Anatomical reference point
Pk, Pk(t): Dissection point
Ps(t): Standard dissection process
Matlas: Standard dissection process data
Mpatient: Patient-specific three-dimensional medical image data
M': Dissection process data most suitable to patient
Mfinal: Dissection process data after modifying M' (modified dissection process data)

**Claims**

1. A dissection process estimation device comprising:

   a surgery process case database that stores case data of a surgery process describing a dissection surface corresponding to a dissection site of a dissection target organ and a dissection process providing information about progress of dissection;
   a standard dissection process generation unit that reads case data corresponding to a dissection site designated by a user from the surgery process case database, and generates a standard dissection surface and a standard dissection process based on a plurality of pieces of case data that have been read;

an anatomical reference point extraction unit that extracts an anatomical reference point from patient-specific three-dimensional medical image data; and

a patient-specific dissection process estimation unit that performs matching of the standard dissection surface and the standard dissection process with the patient-specific three-dimensional medical image data, and estimates a patient-specific dissection surface and a patient-specific dissection process.

2. The dissection process estimation device according to claim 1, further comprising:
a dissection point acceptance unit that accepts input of one or a plurality of dissection points to a patient-specific three-dimensional medical image displayed on a display screen.

3. The dissection process estimation device according to claim 1, wherein,
as preprocessing of the matching, the patient-specific dissection process estimation unit performs:

(i) an enlarging or reducing process that corrects difference in a size of an organ captured on the patient-specific three-dimensional medical image data, the difference being caused by difference in an image capturing condition;
(ii) a distortion correction process that corrects difference between a size of the organ that is specific to a patient and a size of a standard organ; and
(iii) all or any one of the processes.

4. The dissection process estimation device according to claim 2, wherein the patient-specific dissection process estimation unit estimates the patient-specific dissection process based on the standard dissection process, the anatomical reference point extracted from the patient-specific three-dimensional medical image data, and the dissection points accepted from the user.

5. The dissection process estimation device according to claim 4, wherein the patient-specific dissection process estimation unit calculates the dissection surface passing by near the one or a plurality of dissection points designated by the user as a solution of a minimization problem that assumes positions of the dissection points to be a constraint.

6. The dissection process estimation device according to claim 2, wherein, when the standard dissection surface and the standard dissection process are generated by combining individual pieces of the case data stored in the surgery process case database, the standard dissection process generation unit performs weighting of the pieces of case data to be combined to obtain a combination most approximating the anatomical reference point extracted from the patient-specific three-dimensional medical image data and the dissection points accepted from the user.

7. The dissection process estimation device according to claim 1, wherein
the dissection surface is constituted by a partial curved surface generated in accordance with individual progress of dissection, and
the dissection process provides an order of dissection corresponding to the partial curved surface.

8. The dissection process estimation device according to claim 4, wherein the patient-specific dissection process estimation unit estimates a partial curved surface passing by any of the dissection points based on the standard dissection surface and the standard dissection process.

9. The dissection process estimation device according to claim 8, wherein the patient-specific dissection process estimation unit estimates the partial curved surface by using an anatomical structure of the anatomical reference point.

10. The dissection process estimation device according to claim 2, wherein the dissection point acceptance unit accepts input of the one or a plurality of dissection points, and simultaneously, accepts a dissection order of the inputted dissection points.

11. The dissection process estimation device according to claim 10, wherein the patient-specific dissection process estimation unit associates positions and input order of the one or a plurality of dissection points inputted to the patient-specific three-dimensional medical image data with vertexes of each mesh constituting a standard organ, and records the positions and the input order in the surgery process case database.

12. The dissection process estimation device according to claim 2, wherein the patient-specific dissection process estimation unit associates the standard dissection surface with vertex information of the patient-specific three-dimensional medical image data corresponding to the dissection points designated by the user.

**13.** The dissection process estimation device according to claim 2, wherein the patient-specific dissection process estimation unit associates the patient-specific dissection process and vertex information of the patient-specific three-dimensional medical image corresponding to the dissection points designated by the user with vertex information of a standard organ.

**14.** A dissection process estimation device comprising:

a surgery process case database that stores case data of a surgery process describing a dissection surface corresponding to a dissection site of a dissection target organ and a dissection process providing information about progress of dissection;
a standard dissection process generation unit that reads case data corresponding to a dissection site designated by a user from the surgery process case database, and generates a standard dissection surface and a standard dissection process based on a plurality of pieces of case data that have been read;
an anatomical reference point extraction unit that extracts an anatomical reference point from patient-specific three-dimensional medical image data; and
a patient-specific dissection process estimation unit that performs matching of the standard dissection surface and the standard dissection process with the patient-specific three-dimensional medical image data, and estimates a patient-specific dissection surface and a patient-specific dissection process, wherein
the information about progress of dissection is expressed by using a set V of vertexes of each mesh representing the dissection target organ and an element set E,
the set V is expressed, by a vertex vector vi constituted by a plurality of pieces of information provided to each of the vertexes, as

$$V = \{v0, v1, ..., vn\text{-}1\},$$

the vertex vector vi is expressed, by using vertex coordinates (x, y, z) providing information of each of the vertexes, a time stamp (time), an anatomical structure label (label), first attribute information (isFreezed), second attribute information (isCutpath), and third attribute information (isSurface), as

$$vi = (x, y, z, time, label, isFreezed, isCutpath, isSurface),$$

the time stamp (time) shows in what order a corresponding vertex is dissected,
the anatomical structure label (label) is a value showing an anatomical structure,
the first attribute information (isFreezed) shows whether the corresponding vertex is a fixed point or a free point,
the second attribute information (isCutpath) shows whether or not the corresponding vertex is presented on the dissection surface,
the third attribute information (isSurface) shows whether the corresponding vertex is on a surface or inside of the dissection target organ, and
the element set E is a set of combinations of vertexes providing individual minimum elements constituting each of the each mesh.

**15.** A dissection process navigation system comprising:

a surgery process case database that stores case data of a surgery process describing a dissection surface corresponding to a dissection site of a dissection target organ and a dissection process providing information about progress of dissection;
a standard dissection process generation unit that reads case data corresponding to a dissection site designated by a user from the surgery process case database, and generates a standard dissection surface and a standard dissection process based on a plurality of pieces of case data that have been read;
an anatomical reference point extraction unit that extracts an anatomical reference point from patient-specific three-dimensional medical image data;
a patient-specific dissection process estimation unit that performs matching of the standard dissection process with the patient-specific three-dimensional medical image data, and estimates a patient-specific dissection process; and

a display device that displays the patient-specific dissection process estimated in the patient-specific dissection process estimation unit.

## FIG.1

SURGERY PROCESS CASE DATABASE — 130

PATIENT-SPECIFIC THREE-DIMENSIONAL MEDICAL IMAGE DATA STORAGE DEVICE (e.x.CT DATA, MRI DATA) — 120

110

STANDARD DISSECTION PROCESS GENERATION UNIT — 111

$p_s(t)$

$M_{atlas}$

$p_a$

ANATOMICAL REFERENCE POINT EXTRACTION UNIT — 112

$p_a$

$M_{patient}$

PATIENT-SPECIFIC DISSECTION PROCESS ESTIMATION UNIT — 113

100

DISPLAY DEVICE — 140

FIG.2

FIG.3A    $t = 0$

FIG.3B    $t = 1$

FIG.3C    $t = 2$

FIG.3D    $t = 3$

DISSECTION

FIG.4A

$M_{atlas}$

MATCHING

FIG.4B

$M_{patient}$

FIG.4C

$M'$

FIG.5

FIG.6A

20

60A

61A

Pa(ARTERY)

FIG.6B

20

61A(50)

Pa(VEIN)

60B

61B(50)

FIG.6C

20

50

Pa
(BLOOD VESSEL OF
SITE TO BE DISSECTED
(TRANSPARENT VIEW))

Pa(VEIN)

FIG.7

FIG.8B

31

DISSECTION

20

$M'$

FIG.8C 41

20

$\mathbf{p}_k$

$\mathbf{p}_k$

$\mathbf{p}_k$

$M_{final}$

CONSTRAINT CONDITION

FIG.8A

20

$\mathbf{p}_k$

$\mathbf{p}_k$

$\mathbf{p}_k$

FIG.9

FIG.10

SURGERY PROCESS CASE DATABASE 130

STANDARD DISSECTION PROCESS GENERATION UNIT 111

$P_k(t)$

$p_s(t)$  $M_{atlas}$

110B

DISSECTION POINT ACCEPTANCE UNIT 114B

$P_k(t)$

PATIENT-SPECIFIC DISSECTION PROCESS ESTIMATION UNIT 113B

PATIENT-SPECIFIC THREE-DIMENSIONAL MEDICAL IMAGE DATA STORAGE DEVICE (e.x.CT DATA, MRI DATA) 120

$p_a$  ANATOMICAL REFERENCE POINT EXTRACTION UNIT 112

$p_a$
$M_{patient}$

DISPLAY DEVICE 140

300

FIG.11B

31

DISSECTION

20

$M'$

FIG.11C

51

20

Pk(1)

Pk(2)

Pk(0)

$M_{final}$

CONSTRAINT CONDITION

FIG.11A

20

Pk(2)

Pk(1)

Pk(0)

FIG.12

FIG.13

Pa
(TUMOR)

Pa
(TUMOR)

Pa
(ARTERY)

CASE OF DISSECTION OF
RIGHT INFERIOR LOBE

FIG.14

Pa(ARTERY)

Pa (TUMOR)

CASE OF DISSECTION OF
LEFT INFERIOR LOBE

**INTERNATIONAL SEARCH REPORT**

| International application No. |
| --- |
| PCT/JP2017/023556 |

| A. CLASSIFICATION OF SUBJECT MATTER |
| --- |
| *A61B6/03*(2006.01)i, *A61B34/10*(2016.01)i, *A61B34/20*(2016.01)i |

According to International Patent Classification (IPC) or to both national classification and IPC

| B. FIELDS SEARCHED |
| --- |
| Minimum documentation searched (classification system followed by classification symbols)<br>A61B6/03, A61B34/10, A61B34/20 |

Documentation searched other than minimum documentation to the extent that such documents are included in the fields searched

| Jitsuyo Shinan Koho | 1922–1996 | Jitsuyo Shinan Toroku Koho | 1996–2017 |
| Kokai Jitsuyo Shinan Koho | 1971–2017 | Toroku Jitsuyo Shinan Koho | 1994–2017 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| X<br>A | JP 2015-517327 A  (Siemens Product Lifecycle Management Software Inc.), 22 June 2015 (22.06.2015), paragraphs [0012] to [0081]; fig. 1 to 5 & US 2013/0297265 A1 paragraphs [0016] to [0084]; fig. 1 to 5 & WO 2013/165883 A1    & EP 2844178 A1 & CN 104271067 A | 1-13,15<br>14 |
| A | WO 2015/135056 A1  (SYNAPTIVE MEDICAL (BARBADOS) INC.), 17 September 2015 (17.09.2015), entire text; all drawings & JP 2017-514637 A    & US 2016/0354155 A1 & EP 3116435 A1    & CA 2942417 A1 & CN 106232047 A    & KR 10-2017-0034349 A | 1-15 |

| ☒ Further documents are listed in the continuation of Box C. | ☐ See patent family annex. |
| --- | --- |

* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>05 September 2017 (05.09.17) | Date of mailing of the international search report<br>19 September 2017 (19.09.17) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2017/023556

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2003-141566 A  (Kansai Technology Licensing Organization Co., Ltd.), 16 May 2003 (16.05.2003), entire text; all drawings (Family: none) | 1-15 |
| A | WO 2010/021309 A1  (Nara Institute of Science and Technology), 25 February 2010 (25.02.2010), paragraphs [0188] to [0243] (Family: none) | 1-15 |
| A | WO 2010/113690 A1  (Nara Institute of Science and Technology), 07 October 2010 (07.10.2010), paragraphs [0163] to [0190] (Family: none) | 1-15 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013154037 A
- JP 2014176425 A